# EUROPEAN PATENT APPLICATION

(11) **EP 4 477 217 A1**
(43) Date of publication of application: **18.12.2024**
(21) Application number: 22922565.1
(22) Date of filing: 22.12.2022
(51) Int. Cl.: A61K 31/155, A61P 31/04, A61K 47/40, A61P 31/12

(54) **LONG-LASTING ANTIMICROBIAL COMPOSITIONS CONTAINING CATIONIC ACTIVE AGENTS AND ANIONIC EXCIPIENTS AND USES**

(30) Priority: 28.01.2022 BR 102022001653
(71) Applicant: Universidade Federal De Minas Gerais - UFMG, 31270-901 Belo Horizonte (BR); 2Heal Industria e Comercio de Produtos Quimicos Para uso Industrial Ltda, 13211-698 Jundiaí (BR)
(72) Inventor: MOISES IWAMIZU SILVA, Renata, 13211-685 Jundiai (BR); SINISTERRA MILLÁN, Rubén Dario, 31270-901 Belo Horizonte (BR); CORTÉS SEGURA, María Esperanza, 31270-901 Belo Horizonte (BR); OURIVES BOMFIM FILHO, Llucius Flavius, 31270-901 Belo Horizonte (BR)
(74) Representative: Patentree
(86) International application number: PCT/BR2022/050521
(87) International publication number: WO 2023/141689

(57) **Abstract**

This invention refers to long-lasting antibacterial compositions with a broad spectrum of activity, including bactericidal, bacteriostatic, virucidal, virustatic, germicidal, algicidal, fungicidal, and fungistatic activities. These compositions can be used for the production of topical formulations or for environmental treatment, surface disinfection, or dental use. The composition can be in the form of a solution, a moist gel, xerogel, aerosol, spray, polymeric system, or solid state. Specifically, the invention refers to compositions, such as aqueous or alcoholic solutions, or even in gel or powder form, with antibacterial activity. These compositions contain a cationic active (chlorhexidine digluconate, quaternary ammonium, or cetil pyridine) nanoencapsulated in cyclodextrin (alpha-cyclodextrin, beta-cyclodextrin, gamma-cyclodextrin, hydroxymethyl beta-cyclodextrin, or hydroxypropyl beta-cyclodextrin), which may optionally include alkyl or aryl group insertions, at a molar ratio of 1:1 to 1:4. These compositions also include anionic excipients, as well as humectants, stabilizers, sequestering agents, surfactants, preservatives, or thickeners. Preferably, they include the anionic surfactant sodium lauryl sulfate or the anionic thickener carbomer.

## Description

The present invention relates to compositions with long-lasting and broad-spectrum antimicrobial activity, including bactericidal, bacteriostatic, virucidal, virostatic, germicidal, algicidal, fungicidal, and fungistatic activities. The compositions can be used for the production of formulations for topical use or for environmental treatment, surface hygiene, or dental use. The composition can be in solution, moist gel, xerogels, aerosols, sprays, polymeric systems, and solid state. In particular, the invention refers to compositions as an aqueous solution or alcoholic solution or even in the form of gel or powder with antimicrobial activity. The compositions contain a cationic active (chlorhexidine digluconate, quaternary ammonium, or cetylpyridinium) nanoencapsulated in cyclodextrin (alpha-cyclodextrin, beta-cyclodextrin, gamma-cyclodextrin, hydroxyethyl-beta-cyclodextrin, or hydroxypropyl-beta-cyclodextrin, which may optionally contain alkyl or aryl group insertions) in a molar ratio of 1:1 to 1:4. These compositions further comprise anionic excipients, as well as humectants, stabilizers, sequestering agents, surfactants, preservatives, or thickeners. Preferably, they comprise the anionic surfactant sodium lauryl sulfate or the anionic thickener carbomer.

The present invention refers to the obtaining of solids, gels, or liquids containing an inclusion compound of cationic actives such as chlorhexidine salts and cyclodextrin in various molar ratios for antifungal, antibacterial, algicidal, germicidal, and antiviral applications, including SARS-COV, combining the sustained release system of the inclusion complex like chlorhexidine (CHX:CD) due to the virucidal activity of chlorhexidine especially enhanced with nanoencapsulation in molar ratios of 1:1 to 1:4.

Chlorhexidine and its salts are well known for their antimicrobial activity and have been used in aqueous-based oral compositions to combat plaque formation and cavities caused by bacteria in the oral cavity. Chlorhexidine is active against a wide range of Gram-positive and Gram-negative bacteria, as well as yeasts, fungi, and viruses (Denadai A.M.L.; Teixeira K.I.; Santoro M.M.; Pimenta A.M.C.; Cortés M.E.; Sinisterra R.D. Supramolecular self-assembly of β-cyclodextrin: an effective carrier of the antimicrobial agent chlorhexidine. Carbohydr. Res. 342, 2286-2296, 2007).

It is well known that chlorhexidine is an antiseptic agent for skin and mucous membranes, usually produced as diacetate or digluconate salts, commonly used as a disinfectant in areas contaminated by microorganisms such as fungi, bacteria, and viruses (Bui L.N.; Swan J.T.; Perez K.K.; Johnson M.L.; Chen H.; Colavecchia A.C.; Rizk E.; Graviss E.A. Impact of chlorhexidine bathing on antimicrobial utilization in surgical intensive care unit. J. Surg. Res. 250, 161-171, 2020). Additionally, it can be found in many personal hygiene products such as toothpaste, cosmetic products, lubricants, or as a preservative agent.

However, there are reports of allergic reactions to chlorhexidine with a wide spectrum of manifestations from mild skin rashes to anaphylactic shock (Moka E.; Argyra E.; Siafaka I.; Vadalouca A. Chlorhexidine: Hypersensitivity and anaphylactic reactions in the perioperative setting. J. Anaesthesiol. Clin. Pharmacol. 31, 145-148, 2015). Some of the reactions to chlorhexidine have been associated with the topical application of this agent on open wounds or oral mucosa as a result of usage procedures.

A strategic possibility to avoid allergic reactions to chlorhexidine is the sustained release of this compound using cyclodextrin as proposed in the present invention. In the state of the art, some documents describe the use of chlorhexidine in multiple drug candidate formulations for various biological applications or material use.

The state of the art shows that toothpaste can be used as a means to deliver antimicrobial agents. A constant search for improvements in existing dentifrices has resulted in the addition of a number of chemicals to dentifrices, mainly antimicrobials, that offer the potential for adjunctive benefit to gingival health, such as chlorhexidine. It can be incorporated into toothpaste to enhance antimicrobial action or be a mouthwash for post-brushing use. Chlorhexidine is a cationic bis-biguanide with anti-plaque action and broad antibacterial activity against cariogenic bacteria. The property of substantivity, i.e., the prolonged action capacity of chlorhexidine against oral microorganisms. However, to date, dentifrices containing chlorhexidine are not widely commercially available due to its reaction with sodium lauryl sulfate (SLS) surfactant present in toothpastes with the possibility of reducing the antimicrobial activity of both actives (Venu V. et al. Comparative evaluation of antibacterial property and substantivity of chlorhexidine-containing dentifrices with sodium lauryl sulfate and Tween as surfactants: An in vivo study. Indian Journal of Dental Research, vol. 24, no. 4, 2013, p. 521. Accessed 14 Oct. 2020).

The literature reports that the use of solutions containing sodium lauryl sulfate before or after the use of chlorhexidine reduces its antimicrobial action by at least 4 hours (Elkerbout T.A Slot D.E. Bakker E.W.]P. Van der Weijden G.A. Chlorhexidine mouthwash and sodium lauryl sulfate dentifrice: do They mix effectively or interfere? Int. Dent. Hygiene 14, 2016, 42-52). This disadvantage in combined use creates difficulty in applying mouthwashes containing chlorhexidine or its salts after tooth brushing, as toothpastes contain sodium lauryl sulfate in their formulations. The present technology brings the advantage of the joint application of sodium lauryl sulfate along with the inclusion compound cyclodextrin without losing antimicrobial action.

There are also several patent documents in the state of the art showing that anionic substances in formulations are incompatible with cationic actives, as reported in patent document WO 2016/059653 of 2014, where the use of sulfonated azo dyes such as azorubine, however, has the disadvantage of causing precipitation of chlorhexidine in hydroalcoholic solutions, drastically reducing its antimicrobial activity. For this reason, it is often necessary to prepare the antiseptic solution immediately prior to use in order to avoid rapid precipitation (and therefore deactivation) of the components.

Thus, the present technology aims to solve this problem in the state of the art where the inclusion compounds of chlorhexidine: cyclodextrins salts show high and long-lasting antimicrobial activity and efficiency even in the presence of anionic stabilizers or surfactants such as sodium lauryl sulfate due to the protection by molecular encapsulation of chlorhexidine using cyclodextrins.

Studies presented in the literature show that the entropic and enthalpic parameters of the interaction of the carbomer-based gel and chlorhexidine are affected by the insertion of molecules such as surfactants, emollients, or additives (Buckton G, Tawburic S. Drug release form gel bases: a case for enthalpy-entropy compensation. Journal of Controlled Release 20, 1992, 29-36). With this, the possible loss of chlorhexidine activity by the action of carbomer due to the electrostatic interaction between the salt and the polymer can be avoided with the use of protective or encapsulating stabilizing groups, such as cyclodextrins and their structural variations that act as protective groups for chlorhexidine and reduce its interaction with carbomer.

Thus, the present invention describes antimicrobial compositions that can be used to produce transparent gels using carbomers that contain the active chlorhexidine or its salts encapsulated with cyclodextrin or its structural variations in various molar proportions without the loss of the antimicrobial property of chlorhexidine.

The extensive use of carbomer as the main gel thickener for commercial use refers to obtaining transparent gels and a better tactile sensation when used. Therefore, the study of the stability of products containing the polymer that meet the requirements with a broad-spectrum antiseptic active such as chlorhexidine salts, especially nanoencapsulated in cyclodextrins, present themselves as optimized application systems to meet consumer demands with antimicrobial activity and positive sensory experience.

Patent document RU2585384C2 of 2014, titled "Soluble pharmaceutical composition based on fipronil and yuvemon for treating arachnoentomoses," refers to the composition of a multicomponent drug of fipronil, yuvemon, prednisolone, chlorhexidine or myristin, lidocaine, dimethyl sulfoxide, 2-hydroxypropyl-β-cyclodextrin, and as solvents water and alcohol, including isopropyl or ethyl alcohol and propylene glycol in a volume ratio of 1:3 to 1:5 (% w/w) with antiparasitic activity and therapeutic efficacy for treating arachnoentomosis.

The patent document US5776430, from 1994, titled "Topical antimicrobial cleanser containing chlorhexidine gluconate and alcohol," refers to the combination of 0.65 to 0.85% (w/w) of chlorhexidine gluconate, denatured ethyl alcohol, and cetyl lactate, producing a highly effective topical antimicrobial cleanser with immediate, persistent, and residual bactericidal activity. It also refers to a low-temperature manufacturing process for the production of chlorhexidine salts in alcohol-based formulations.

The application of gels with chlorhexidine salts is observed in dental applications. Savitha and collaborators (2019) presented a study indicating that the inclusion of chitosan in a gel containing 2% chlorhexidine increases antimicrobial efficacy against the bacterium *Enterococcus faecalis,* making this material a candidate for use as an intracanal medicament for failed endodontic procedures (Savitha, A.; SriRekha, A.; Vijay, R.; Asheija, Champa, C.; Jaykumar, T. An in vivo comparative evaluation of antimicrobial efficacy of chitosan, chlorhexidine gluconate gel and their combination as an intracanal medicament against Enterococcus faecalis in failed endodontic cases using real-time polymerase chain reaction (qPCR). Saudi Dental Journal, 31, 360-366, 2019).

The use of chlorhexidine salts at a concentration of 0.12% is quite common in commercial mouthwashes. However, their use is suggested for up to 14 days and after 30 minutes of brushing. The first point refers to the change in tooth color, from white to yellowish, due to the continuous use of chlorhexidine at high concentrations; another point concerns the loss of antimicrobial properties of chlorhexidine salt when in contact with certain substances present in toothpaste, such as surfactants. Additionally, there is the characteristic taste of chlorhexidine salts in solution, which makes mouthwashes unpleasantly bitter.

The present invention presents studies on anionic surfactants that show the maintenance of the activity of chlorhexidine digluconate salt, encapsulated in cyclodextrin, to reduce the bitterness of solutions containing chlorhexidine salts when compared to the same concentration of free chlorhexidine digluconate in solution and the possibility of using the product for more than 30 consecutive days without causing tooth discoloration.

Some of the halogenated antimicrobial compounds specially studied, with broad spectrum antimicrobial activity, and which are useful in the compositions of the present invention include 1,1'-hexamethylene bis(5-*(p-chlorophenyl)* biguanide), commonly known as chlorhexidine and its salts, for example, with hydrochloric, acetic, and gluconic acids. The digluconate salt is highly water-soluble, about 70% in water, and the diacetate salt has a solubility of about 1.8% in water.

Other useful biguanide compounds include Cosmoci^{®} CQ^{®} and Vantocil^{®} IB which contain poly(hexamethylene biguanide) hydrochloride. Other useful cationic antimicrobial agents include bis-biguanide alkanes. The water-soluble salts usable thereof include chlorides, bromides, sulfates, alkylsulfonates such as methylsulfonate and ethylsulfonate, phenylsulfonates such as p-methylphenylsulfonates, nitrates, acetates, gluconates, and similar compounds.

Examples of suitable bis-biguanide compounds include chlorhexidine; 1,6-bis-(2-ethylhexylbiguanido-hexane) dichloride; 1,6-di-(N1,N1'-phenyldiguanido-N5,N5')-hexane tetrahydrochloride; 1,6-di-(N1,N1'-phenyl-N1,N1'-methyldiguanido-N5,N5')-hexane dichloride; 1,6-di-(N1,N1'-o-chlorophenyldiguanido-N5,N5')-hexane dichloride; 1,6-di-(N1,N1'-2,6-dichlorophenyldiguanido-N5,N5')-hexane dichloride; 1,6-di-[N1,N1'-Beta-(p-methoxyphenyl)diguanido-N5,N5']-hexane dichloride; 1,6-di-(N1,N1'-Alphamethyl-beta-phenyldiguanido-N5,N5')-hexane dichloride; 1,6-di-(N1,N1'-p-nitrophenyldiguanido-N5,N5')-hexane dichloride; Omega'-di-(N1,N1'-phenyldiguanido-N5,N5')-di-dichloride n-propyl ether; Omega'-di-(N1,N1'-p-chlorophenyldiguanido-N5,N5 ')-tetrahydrochloride di-n-propyl ether; 1,6-di-(N 1, N 1 '-2,4-dichlorophenyldiguanido-N5, N5')-hexane tetrahydrochloride; 1,6-di-(N1,N1'-p-methylphenyldiguanido-N5,N5')-hexane dichloride; 1,6-di-(N1,N1'-2,4,5-trichlorophenyldiguanido-N5,N5')-hexane tetrahydrochloride; 1,6-di-[N1,N1'-Alpha-(p-chlorophenyl)ethyldiguanido-N5,N5']-hexane dichloride; Omega.: Mega-di-(N 1, N 1 '-p-chlorophenyldiguanido-N5, N5) m-xylene dichloride; 1,12-di-(N1,N1'-p-chlorophenyldiguanido-N5,N5')-dodecane dichloride; 1,10-di-(N1,N1'-phenyldiguanido-N5,N5')-decane tetrahydrochloride; 1,12-di-(N1,N1'-phenyldiguanido-N5,N5') dodecane tetrahydrochloride; 1,6-di-(N1, N1'-o-chlorophenyldiguanido-N5,N5')-hexane dichloride; 1,6-di-(N1,N1'-p-chlorophenyldiguanido-N5,N5')-hexane tetrahydrochloride; Ethylene bis(1-tolyl biguanide); Ethylene bis(p-tolyl biguanide); Ethylene bis(3,5-dimethylphenyl biguanide); Ethylene bis(p-tert-amylphenyl biguanide); Ethylene bis(nonylphenyl biguanide); Ethylene bis(phenylbiguanide); Ethylene bis(N-butylphenyl biguanide); Ethylene bis(2,5-diethoxyphenyl biguanide); Ethylene bis(2,4-dimethylphenyl biguanide); Ethylene bis(o-diphenylbiguanide); Ethylene bis(amil-naphthyl mixed biguanide); N-butyl ethylene bis(phenylbiguanide); Trimethylene bis(o-tolyl biguanide); N-butyl trimethylene bis(phenyl biguanide); and the corresponding pharmaceutically acceptable salts of all the above, such as acetates; gluconates; hydrochlorides; bromides; citrates; bisulfites; fluorides; polyalates; N-alkylalkylsarcosinates; phosphites; hypophosphites; perfluorooctanoates; silicates; sorbates; salicylates; maleates; tartarates; fumarates; ethylenediaminetetraacetates; iminodiacetates; cinnamates; thiocyanates; arginates; pyromellitates; tetracarboxybutyrates; benzoates; glutarates; monofluorophosphates; and perfluoropropionates, and mixtures thereof.

The antimicrobial and antiviral agents and their respective inclusion compounds with cyclodextrins in molar ratios of 1:1 to 1:4 or higher preferred molar ratios from this group are 1,6-di-(N1, N1'-phenyldiguanido-N5,N5')-hexane tetrahydrochloride; 1,6-di-(N1,N1'-o-chlorophenyldiguanido-N5,N5')-hexane dichloride; 1,6-di-(N1,N1'-2,6-dichlorophenyldiguanido-N5,N5')-hexane dichloride; 1,6-di-(N1,N1'-2,4-dichlorophenyldiguanido-N5,N5')-hexane tetrahydrochloride; 1,6-di-[N1,N1'-Alpha-(p-chlorophenyl)ethyldiguanido-N5,N5']-hexane dichloride; Omega.: Mega-di-(N1,N1'-p-chlorophenyldiguanido-N5,N5')-m-xylene dichloride; 1,12-di-(N1,N1'-p-chlorophenyldiguanido-N5,N5')-dodecane dichloride; 1,6-di-(N1,N1'-o-chlorophenyldiguanido-N5,N5')-hexane dichloride; 1,6-di-(N1,N1'-p-chlorophenyldiguanido-N5,N5')-hexane tetrahydrochloride; and mixtures thereof; more preferably, 1,6-di-(N1,N1'-o-chlorophenyldiguanido-N5,N5')-hexane dichloride; 1,6-di-(N1,N1'-2,6-dichlorophenyldiguanido-N5,N5')-hexane dichloride; 1,6-di-(N1,N1'-2,4-dichlorophenyldiguanido-N5,N5')-hexane tetrahydrochloride; 1,6-di-[N1,N1'-Alpha-(p-chlorophenyl)ethyldiguanido-N5,N5']-hexane dichloride; Omega.: Mega-di-(N1,N1'-p-chlorophenyldiguanido-N5,N5')-m-xylene dichloride; 1,12-di-(N1,N1'-p-chlorophenyldiguanido-N5,N5')-dodecane dichloride; 1,6-di-(N1,N1'-o-chlorophenyldiguanido-N5,N5')-hexane dichloride; 1,6-di-(N1,N1'-p-chlorophenyldiguanido-N5,N5')-hexane tetrahydrochloride; and mixtures thereof.

A wide variety of quaternary compounds can also be used as antimicrobial and antiviral actives for compositions with cyclodextrins and their inclusion compounds in molar ratios of 1:1 to 1:4 or higher molar ratios of quaternary ammonium: compounds amônio: cyclodextrins of the present invention. Non-limiting examples of useful quaternary compounds include: (1) benzalkonium chlorides and/or substituted benzalkonium chlorides, such as Barquat^{®} (available from Lonza), Maquat^{®} (available from Mason), Variquat^{®} (available from Goldschmidt), and Hyamine^{®} (available from Lonza); (2) short-chain di-(C6-C14) alkyl quaternaries (C1-4 alkyl and/or hydroxyalkyl), such as Bardac^{®} products from Lonza; (3) N-(3-chloroallyl)hexamine chlorides, such as Dowicide^{®} and Dowicil^{®} available from Dow; (4) benzethonium chloride such as Hyamine^{®} 1622 from Rohm & Haas; (5) methylbenzethonium chloride represented by Hyamine^{®} 10× provided by Rohm & Haas; (6) cetylpyridinium chloride, such as Cepacol chloride, available from Merrell Labs. Examples of preferred dialkyl quaternaries include di-(C8-C12) dialkyl dimethylammonium chloride, such as didecyldimethylammonium chloride (Bardac 22) and dioctyl dimethylammonium chloride (Bardac 2050).

The patent document CA1314228 from 1988, titled *"Controlled release agent for cetylpyridinium chloride,"* describes the formulation and controlled release of cetylpyridinium chloride in inclusion compound using cyclodextrin as the encapsulating agent (host). This supports the possibility of utilizing the cationic active cetylpyridinium chloride, present in mouth rinses, associated with anionic excipients, enabling formulations of toothpaste, foams, sprays, or gels, expanding the range of applications and potentially improving their activities.

Cyclodextrins are cyclic oligosaccharides composed of six, seven, or eight glucose units. Due to steric interactions, cyclodextrins (CDs) form a structure resembling a truncated cone with an internal apolar cavity. These are chemically stable compounds that can be selectively modified. Cyclodextrins act as hosts to form complexes with various guests within their cavity. In the specific case discussed, they interact with antimicrobial compounds such as chlorhexidine to obtain compounds in molar ratios of 1:1 to 1:4 of chlorhexidine:cyclodextrins.

The document EP0306455 from 1987, titled *"Cyclodextrin complexes of bis-biguanide hexane compounds,"* provides cyclodextrin complexes of bis-biguanide hexane compounds such as chlorhexidine and its salts, which act to increase the water solubility of chlorhexidine or its salts, mask its bitter taste, and enhance its bioavailability. The cyclodextrin complexes of bis-biguanide hexane compounds are formed by dissolving an excess molar amount of cyclodextrin in deionized water at elevated temperatures and adding chlorhexidine or its salts. The solution is then cooled, frozen, and lyophilized to obtain the complex.

Cyclodextrins have been utilized for solubilizing and encapsulating drugs, perfumes, and fragrances, as described by Zeitli, J., in Chemical Reviews (1998), 98, 1743-1753, and Szeitli, J., in J. Mater. Chem. (1997). According to detailed studies on the toxicity, mutagenicity, teratogenicity, and carcinogenicity of cyclodextrins, described by Rajewski, R. A., and Stella, V., in J. Pharmaceutical Sciences (1996), 85, 1142-1169, they exhibit low toxicity, especially hydroxypropyl β-cyclodextrin, as reported by Szeitli in 2004 (J. Cyclodextrins: Properties and Applications. Drug Invest, 20 Suppl. 4: 11-21, September 2, 2004). Except for some high concentrations causing damage to erythrocytes, these products are generally not harmful to health.

The use of cyclodextrins as food additives has already been authorized in countries such as Japan and Hungary, and for more specific applications in France and Denmark. Moreover, they are obtained from a renewable source of starch degradation. All these characteristics are significant motivations for researching new applications. Practical uses are classified as follows: (1) carriers (solubilizers, stabilizers) for biologically active substances; (2) enzyme mimics; (3) separating agents (for chromatography or batch processes); (4) catalysts and additives and detergents, viscosity modifiers, etc. (L. Szente and J. Szejtli, Adv. Drug Deliv. Rev. 36 (1999), 17).

CDs are moderately soluble in water, methanol, and ethanol, and readily soluble in polar solvents such as dimethyl sulfoxide and N,N-dimethylformamide, N,N-dimethylacetamide, and pyridine. There are numerous research papers in the literature on the effects of increasing water solubility of poorly water-soluble guests using cyclodextrins via inclusion compounds in use (Szeitli, J., Chemical Reviews, (1998), 98, 1743-1753. Szetjli, J., J. Mater. Chem., (1997), 7.575-587). Thus, controlled release systems using various types of high-performance carrier materials have been developed to provide the required amount of antimicrobial, efficiently and accurately, as described in the present technology, based on chlorhexidine: cyclodextrins inclusion compounds in molar ratios of 1:1 to 1:4 or higher, and other cationic actives such as cetylpyridinium chloride and ammonium quaternaries nanoencapsulated in cyclodextrins. The application of chlorhexidine salts against SARS-CoV is due to the observation that these materials exhibit antiviral activity at concentrations above 0.02% w/w (Kampf, G.; Todt, D.; Pfaender, S.; Steinmann, E. Persistence of coronaviruses on inanimate surfaces and their inactivation with biocidal agents. J. Hosp. Infect. 104, 246-251, 2020).

### BRIEF DESCRIPTION OF THE FIGURES

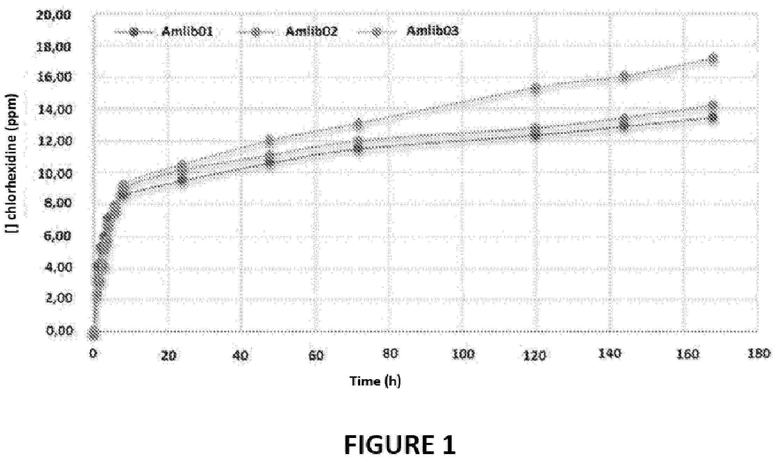

**Figure 1** presents the release curve in tissue impregnated by the foulard method (Amlib01 corresponds to a concentration of 10 g/L; Amlib02 corresponds to a concentration of 7.5 g/L; Amlib03 corresponds to a concentration of 5.0 g/L, in relation to the cationic active nanoencapsulated in cyclodextrins at a molar ratio of 1:1 to 1:4).).

### DETAILED DESCRIPTION OF THE TECHNOLOGY

The present invention relates to compositions with long-lasting and broad-spectrum antimicrobial activity, including bactericidal, bacteriostatic, virucidal, virustatic, germicidal, algicidal, fungicidal, and fungistatic activity. The compositions can be used to produce formulations for topical use or for environmental treatment, surface sanitization, or dental use. The composition can be in solution, wet gel, xerogels, aerosols, sprays, polymeric systems, and solid state. In particular, the invention relates to compositions, such as aqueous solution or alcoholic solution, or even in the form of gel or powder, with antimicrobial activity. The compositions contain a cationic active ingredient (chlorhexidine digluconate, ammonium quaternary, or cetylpyridinium) nanoencapsulated in cyclodextrin (alpha-cyclodextrin, beta-cyclodextrin, gamma-cyclodextrin, hydroxyethyl beta-cyclodextrin, or hydroxypropyl beta-cyclodextrin, which may optionally contain alkyl or aryl group insertions), in the molar ratio of 1:1 to 1:4. These compositions also comprise anionic excipients, as well as including humectants, stabilizers, chelating agents, surfactants, preservatives, or thickeners. Preferably, they comprise the anionic surfactant sodium lauryl sulfate or the anionic thickener carbomer. The long-lasting antimicrobial compositions comprise cationic actives, selected from the group comprising chlorhexidine digluconate, ammonium quaternary, or cetylpyridinium, nanoencapsulated in cyclodextrin, selected from the group comprising alpha-cyclodextrin, beta-cyclodextrin, gamma-cyclodextrin, hydroxyethyl beta-cyclodextrin, and hydroxypropyl beta-cyclodextrin, which may optionally contain alkyl or aryl group insertions, and anionic excipients, as well as humectants, stabilizers, chelating agents, surfactants, preservatives, or thickeners.

The cationic actives can be in the molar ratio of 1:1 to 1:4 in relation to the inclusion compound.

The preservatives can be methylparaben or propylparaben at a concentration of 0.1 to 0.3% w/v.

The anionic pharmaceutical excipients can be the anionic surfactant sodium lauryl sulfate at concentrations between 0.01 and 0.3% v/v; or the thickening agent carbomer at concentrations between 0.1 and 3.0% w/v.

The antimicrobial compositions can be used in the production of sanitizers in aqueous solution or alcoholic solution, in wet gel, xerogels, aerosols, sprays, powders, polymeric systems, and in solid state; in the production of transparent gel; in the production of formulations for topical use; in the production of formulations for environmental treatment and surface sanitization; or in the production of mouthwash and toothpaste.

The pharmaceutical composition may contain clorexidina and its salts such as: Dicloridrato de 1,6-bis-(2-etil-hexilbiguanido-hexano); Tetra-hidrocloreto de 1,6-di-(N1,N1'-fenildiguanido-N5,N5')-hexano; Dicloridrato de 1,6-di-(N1,N1'-fenil-N1,N1'-metildiguanido-N5,N5')-hexano; Dicloridrato de 1,6-di-(N1,N1'-o-clorofenildiguanido-N5,N5')-hexano; Dicloridrato de 1,6-di-(N1,N1'-2,6-diclorofenildiguanido-N5,N5')-hexano; Dicloridrato de 1,6-di-[N1,N1'-Beta-(p-metoxifenil)diguanido-N5,N5']-hexano; Dicloridrato de 1,6-di-(N1,N1'-Alfa-metilbeta-fenildiguanido-N5,N5')-hexano; Dicloridrato de 1,6-di-(N1,N1'-p-nitrofenildiguanido-N5,N5')-hexano; Ômega'-di-(N1,N1'-fenildiguanido-N5,N5')-di-dicloridrato de éter n-propilico; Ômega'-di-(N1,N1'-p-clorofenildiguanido-N5,N5 ')-tetra-hidrocloreto de éter di-n-propilico; Tetra-hidrocloreto de 1,6-di-(N1,N1'-2,4-diclorofenildiguanido-N5,N5')-hexano; Dicloridrato de 1,6-di-(N1,N1'-p-metilfenildiguanido-N5,N5')-hexano; Tetrahidrocloreto de 1,6-di-(N1,N1'-2,4,5-triclorofenildiguanido-N5,N5')-hexano; Dicloridrato de 1,6-di-[N1,N1'-Alfa-(p-clorofenil)etildiguanido-N5,N5']-hexano; Ômega-Mega-di-(N1,N1'-p-clorofenildiguanido-N5,N5) dicloridrato de m-xileno; Dicloridrato de 1, 12-di-(N1, N1 '-p-clorofenildiguanido-N5, N5')-dodecano; 1,10-di-(N1,N1'-fenildiguanido-N5,N5')-tetra-hidrocloreto de decano; 1,12-di-(N1,N1'-fenildiguanido-N5,N5') dodecano tetra-hidrocloreto; Dicloridrato de 1,6-di-(N1, N1'-o-clorofenildiguanido-N5,N5')-hexano; 1,6-di-(N1,N1'-p-clorofenildiguanido-N5,N5')-hexano tetra-hidrocloreto; Etileno bis(1-tolil biguanida); Etileno bis(p-tolil biguanida); Etileno bis(3,5-dimetilfenil biguanida); Etileno bis(p-terc-amilfenil biguanida); Etileno bis(nonilfenil biguanida); Etileno bis(fenilbiguanida); Etileno bis(N-butilfenil biguanida); Etileno bis(2,5-dietoxifenil biguanida); Etileno bis(2,4-dimetilfenil biguanida); Etileno bis(o-difenilbiguanida); Etileno bis(amil-naftil biguanida mista); N-butil etileno bis(fenilbiguanida); Bis(o-tolil biguanida) de trimetileno; N-butil trimetileno bis(fenil biguanida) and the corresponding pharmaceutically acceptable salts of all the above such as acetates; gluconates; chlorides; bromides; citrates; bisulfites; fluorides; polyalates; N-alkylalkylsarcosinates; phosphites; hypophosphites; perfluorooctanoates; silicates; sorbates; salicylates; maleates; tartrates; fumarates; ethylenediaminetetraacetates; iminodiacetates; cinnamates; thiocyanates; arginates; pyrromelites; tetracarboxybutyrates; benzoates; glutarates; monofluorophosphates; and perfluoropropionates and their mixtures.

The antimicrobial and antiviral agents and their respective inclusion compounds with cyclodextrins, in molar ratios between 1:1 and 1:4, are selected from the group comprising tetrahydrochloride of 1,6-di-(N1, N1'-phenyldiguanido-N5,N5')-hexane; Dihydrochloride of 1,6-di-(N1,N1'-o-chlorophenyldiguanido-N5,N5')-hexane; Dihydrochloride of 1,6-di-(N1,N1'-2,6-dichlorophenyldiguanido-N5,N5')-hexane; Tetrahydrochloride of 1,6-di-(N1,N1'-2,4-dichlorophenyldiguanido-N5,N5')-hexane; Dihydrochloride of 1,6-di-[N1,N1'-.Alpha.-(p-chlorophenyl)ethyldiguanido-N5, N5']-hexane; .Omega. Dihydrochloride of Mega.'di-(N1 ,N1 '-p-chlorophenyldiguanido-N5,N5 ')-m-xylene; Dihydrochloride of 1,12-di-(N1,N1'-p-chlorophenyldiguanido-N5,N5') dodecane; Dihydrochloride of 1,6-di-(N1,N1'-o-chlorophenyldiguanido-N5,N5')-hexane; 1,6-di-(N1,N1'-p-chlorophenyldiguanido-N5,N5')-hexane tetrahydrochloride; and mixtures thereof; more preferably, dihydrochloride of 1,6-di-(N1,N1'-o-chlorophenyldiguanido-N5,N5')-hexane; Dihydrochloride of 1,6-di-(N1,N1'-2,6-dichlorophenyldiguanido-N5,N5')-hexane; Tetrahydrochloride of 1,6-di-(N1,N1'-2,4-dichlorophenyldiguanido-N5,N5 ')-hexane; Dihydrochloride of 1,6-di-[N1,N1'-.Alpha.-(p-chlorophenyl)ethyldiguanido-N5, N5']-hexane; . Omega.-Mega. 'di-(N1,N1'-p-chlorophenyldiguanido-N5,N5 ')-m-xylene; Dihydrochloride of 1,12-di-(N1,N1'-p-chlorophenyldiguanido-N5,N5') dodecane; Dihydrochloride of 1,6-di-(N1,N1'-o-chlorophenyldiguanido-N5,N5')-hexane; 1,6-di-(N1,N1'-p-chlorophenyldiguanido-N5,N5')-hexane tetrahydrochloride; and mixtures thereof.

The cationic agents that can be included in the formulation may include variations of the pyridinyl group such as: 2-acetylpyridine, 3-acetylpyridine, 4-acetylpyridine, 4-iodopyridine, 4-methylpyridine, 4-aminopyridine, 4-chloropyridine, 4-fluoropyridine, 4-bromopyridine, 4-hydroxypyridine, 4-mercaptopyridine, 3-aminopyridine, 3-bromopyridine, 3-iodopyridine, 3-fluoropyridine, 3-chloropyridine, 3-methylpyridine, 2-acetylpyridine, 3-acetylpyridine, 4-acetylpyridine, 4-acetyl-2-chloropyridine, 3-acetyl-2-chloropyridine, dipyridin-4-ylmethanone, 1-(4-quinolinyl)ethanone, pyridin-2-yl(pyridin-4-yl)methanone, 1-(2-fluoro-6-(pyrrolidin-1-yl)pyridin-4-yl)ethanone, (2E)-4,4,4-trifluoro-3-hydroxy-1-(4-pyridinyl)-2-buten-1-one, 1,3-bis(1-oxo-pyridin-4-yl)-propenone, dipyridin-4-ylmethanone, cetylpyridinium chloride, cetylpyridinium bromide, cetylpyridinium iodide, 2,4-dioxo-4-pyridin-4-ylbutanoic acid, 2-(2-chlorophenyl)-1-(4-pyridinyl)ethanone; besides, quaternary ammonium salts containing aliphatic or aromatic chains ranging from 1 to 4 substituent groups such as: ammonium formate, ammonium chloride, tetramethylammonium borohydride, tetramethylammonium fluoride, tetramethylammonium chloride, tetramethylammonium iodide, tetrabutylammonium fluoride, tetrabutylammonium chloride, tetrabutylammonium bromide, tetrabutylammonium iodide, tetrabutylammonium nitrate, chlorocholine chloride, benzylmethylammonium chloride, benzyltrimethylammonium bromide, benzyltrimethylammonium iodide, tetraethylammonium nitrate, tetraethylammonium chloride, benzyl-octadecyl-methyl-propylammonium bromide, trimethyloctylammonium chloride, hexyltrimethylammonium bromide, N,N-dimethyldodecylamine N-oxide, tributylmethylammonium chloride, trimethylcetyl-ammonium bromide, benzylhexadecyltrimethylammonium bromide, dodecyltrimethylammonium chloride, dodecyltrimethylammonium bromide, dodecyltrimethylammonium iodide, tris(2-hydroxyethyl)methylammonium methyl sulfate, tetrabutylammonium chlorohydrate, allyl-benzyl-diethylammonium bromide, N-[2-(dimethylnitroil)ethyl]dodecanamide, trimethyl-tetradecylammonium chloride, trimethyl-tetradecylammonium bromide, trimethyl-tetradecylammonium iodide, N, N-dimethyl-N-[(3,5,5,8,8-pentamethyl-5,6,7,8-tetrahydronaphthalen-2-yl)methyl]-1-dodecanamine, N,N-dimethyl-N-[(3,5,5,8,8-pentamethyl-5,6,7,8-tetrahydronaphthalen-2-yl)methyl]-1-dodecanamine bromide, N,N-dimethyl-N-[(3,5,5,8,8-pentamethyl-5,6,7,8-tetrahydronaphthalen-2-yl)methyl]-1-dodecanamine iodide, and N,N-dimethyl-N-[(3,5,5,8,8-pentamethyl-5,6,7,8-tetrahydronaphthalen-2-yl)methyl]-1-dodecanamine nitrate.

The present technology can be better understood through the following non-limiting examples.

### Example 1 - Pharmaceutical composition preparation processes

The process of preparing the gel with carbopol was achieved by adding AMP (aminomethyl propanol) in the appropriate amount to an aqueous solution of carbopol at concentrations between 0.1 and 3.0% w/v.

The process of preparing solutions with inclusion compounds was carried out by adding the chlorhexidine salt to an aqueous solution containing a cyclodextrin encapsulant (hydroxypropyl beta-cyclodextrin, alpha-cyclodextrin, beta-cyclodextrin, gamma-cyclodextrin) at 60°C, maintaining the molar ratios at 1:1, 1:2, 1:3, or 1:4, respectively, following the procedure reported in the literature (CORTÉS et al., The chlorhexidine: β-Cyclodextrin Inclusion compounds: preparation, characterization and microbial evaluation. Journal of Inclusion Phenomena and Macrocyclic Chemistry, 40, 2001, 297-302).

The aqueous or alcoholic solution at an appropriate concentration of the inclusion compound, containing surfactant or emulsifier also at concentrations between 0.01% and 0.3% w/v for preparation, was added to the gel with constant stirring. The addition of the solution containing the active ingredient was performed after cooling the gel to a temperature between 15°C and 35°C in order not to modify the properties of each chemical formulation.

When necessary, methylparaben or propylparaben at a concentration of 0.1% w/v was used as a preservative to prevent fungal contamination during product storage.

### Example 2 - Preparation of antiseptic cosmetic formulations using 1:1 to 1:4 inclusion compounds of chlorhexidine:cyclodextrins salts and pharmaceutical excipients, including anionic surfactants

Long-lasting antimicrobial pharmaceutical compositions for topical use and surface hygiene or cosmetic use were prepared using the following tabulated formulations (Tables 1 to 3).

**Table 1 - Formulation 1 Data.**

| **Compound** | **Amount added** |
|---|---|
| Distilled water | q.s.p |
| Glycerin | 0,02 % (w/v) |
| Cocamidopropyl betaine | 0,05 % (w/v) |
| Cocamide DEA | 0,05 % (w/v) |
| Sodium laureth sulfate | 1,00 % (w/v) |
| Fragrance (black cherry) | 0,10 % (w/v) |
| Polyquaternium-7 | 0,05 % (w/v) |
| Citric acid | 0,01 % (w/v) |
| Imidazolidinyl | 0,10 % (w/v) |
| CHX:CD | 0,10 % (w/v) |

**Table 2 - Formulation 2 Data**

| **Compound** | **Amount added** |
|---|---|
| Distilled water | q.s.p |
| Glycerin | 0,02 % (w/v) |
| Cocamidopropyl betaine | 0,05 % (w/v) |
| Cocamide DEA | 0,05 % (w/v) |
| Sodium laureth sulfate | 1,00 % (w/v) |
| Fragrance (black cherry) | 0,10 % (w/v) |
| Polyquaternium-7 | 0,05 % (w/v) |
| Citric acid | 0,01 % (w/v) |
| Imidazolidinyl | 0,10 % (w/v) |
| CHX:CD | 0,06 % (w/v) |

**Table 3 - Formulation 3 Data**

| **Compound** | **Amount added** |
|---|---|
| Distilled water | q.s.p |
| Glycerin | 0,02 % (w/v) |
| Cocamidopropyl betaine | 0,05 % (w/v) |
| Cocamide DEA | 0,05 % (w/v) |
| Sodium laureth sulfate | 1,00 % (w/v) |
| Fragrance (black cherry) | 0,10 % (w/v) |
| Polyquaternium-7 | 0,05 % (w/v) |
| Citric acid | 0,01 % (w/v) |
| Imidazolidinyl | 0,10 % (w/v) |
| CHX:CD | 0,03 % (w/v) |

### Example 3 - Preparation of transparent gel using inclusion compounds of 1:1 up to 1:4 of chlorhexidine:cyclodextrins salts and pharmaceutical excipients, including carbomer

The lotions were prepared by inserting the appropriate amount of carbomer into water for hydration. Then, under stirring, AMP-95 was added until total polymer opening. An aqueous solution containing the desired quantities for the product formulations (Table 4 and 5) was added over the polymer. The solutions were prepared by adding appropriate amounts of glycerin and cosmoguard under constant stirring to an aqueous solution of inclusion compound. Subsequently, the solution was added, with constant stirring, to the polymer, at a temperature between 10°C and 35°C.

**Table 4 - Formulation 4 Data**

| **Compound** | **Amount added** |
|---|---|
| Distilled water | q.s.p |
| Carbopol | 0,3% (w/v) |
| Glycerin | 1 % (w/v) |
| CHX:CD | 0,02% (w/v) |
| Cosmoguard | 0,4% (w/v) |
| AMP-95 | 0,5 % (w/v) |

**Table 5 - Formulation 5 Data**

| **Compound** | **Amount added** |
|---|---|
| Distilled water | q.s.p |
| Carbopol - 940 | 0,3% (w/v) |
| Glycerin | 1 % (w/v) |
| CHX:CD | 0,05% (w/v) |
| Cosmoguard | 0,4% (w/v) |
| AMP-95 | 0,5 % (w/v) |

### Example 4 - Incorporation of aqueous solution containing the inclusion compound chlorhexidine:cyclodextrins and surfactants in porous wipes or non-woven fabric (TNT)

The solutions applied to the non-woven fabrics were obtained from the formulations presented in Tables 6, 7, and 8. The formulation was carried out by adding the materials directly to the aqueous solution of the inclusion compound in molar ratios between 1:1 and 1:4 of cyclodextrin:chlorhexidine salt with constant stirring and temperature between 10°C and 35°C. To assess the incorporation of the formulated solutions into porous wipe sheets, a piece of porous wipe cut to dimensions of 1.5 cm² was submerged in 10 mL of the solution and left immersed for 1 minute. The material was then placed in an oven at a temperature of 120°C and maintained for 10 minutes.

The pieces were transferred to a container containing 10 mL of distilled water at a temperature of 35°C to evaluate the release time of chlorhexidine into the medium. Aliquots were withdrawn at intervals of 1 minute for the first 10 minutes, then at intervals of 10 minutes for the first hour, hourly after the first hour, and every 24 hours after the first day. All measurements were performed in triplicate.

The release of the material was observed for up to 180 hours, with the type of fabric weight affecting the release of the active ingredient in solution (Figure 1. Release curve TECIDO CREME FOULARD SENAI impregnated by the foulardage methodology (Amlib01 10 g/L; Amlib02 7.5 g/L; Amlib03 5.0 g/L)).

### Example 5 - Physicochemical analysis of the interaction between beta-cyclodextrin, chlorhexidine salt, and surfactant through isothermal nanotitration calorimetry

nteraction studies to assess the stability of the inclusion compound system were conducted through analysis of isothermal nanotitration calorimetry, allowing for the evaluation of the interaction/dissociation constant between beta-cyclodextrin, chlorhexidine digluconate, the inclusion compound formed by the molar ratio of 1:3 (chlorhexidine digluconate:betacyclodextrin), and the surfactant sodium lauryl sulfate.

Interaction studies of the salts with beta-cyclodextrin showed that the strength of interaction with chlorhexidine digluconate is greater than with sodium lauryl sulfate. In adimensional values: 1000 × 10⁶ and 737 × 10⁶, respectively (Table 6).

**Table 6 - Data from the experiment of isothermal calorimetry titration of the interaction between beta-cyclodextrin, chlorhexidine gluconate, and sodium lauryl sulfate.**

| **Interaction** | **Kₐ (10⁶)** | **ΔH (kJ mol⁻¹)** | **ΔS (J mol⁻¹ K⁻¹)** | **n** |
|---|---|---|---|---|
| BCD:clr | 1000 | -52,97 | -5,345 | 0,437 |
| Blend:lss | 1,338 | - 43,65 | -29,0 | 0,845 |
| BCD:lss | 737 | -35,21 | -7,56 | 0,595 |

The calorimetric analyses show that the interaction of chlorhexidine with beta-cyclodextrin is about 1000 times greater than the interaction of sodium lauryl sulfate with beta-cyclodextrin. This indicates that the structure of the inclusion compound is not altered by the presence of the surfactant.

### Example 6 - Physico-chemical analysis of the interaction between the inclusion compound, chlorhexidine digluconate, and carbopol through isothermal nanotitration calorimetry.

Studies of interaction to assess the stability of the inclusion compound system were conducted through analysis of isothermal nanotitration calorimetry, where it was possible to evaluate the interaction/dissociation constant between chlorhexidine digluconate, the inclusion compound formed at a molar ratio of 1:3 (chlorhexidine digluconate: betacyclodextrin), and carbopol already in polymeric structure.

The studies on the interaction of the inclusion compound and free salt in solution showed that there was no significant interaction between the inclusion compound and carbopol when compared to the interaction of chlorhexidine salt in solution (Table 7).

**Table 7 - Data from the experiment of isothermal calorimetry titration of the interaction between inclusion compound, chlorhexidine gluconate, and carbopol.**

| **Interaction** | **Kₐ (10⁶)** | **ΔH (kJ mol⁻¹)** | **ΔS (J mol⁻¹ K⁻¹)** | **n** |
|---|---|---|---|---|
| BCD:clr-carbopol | 0,453 | -1,34 | -7,596 | 0,985 |
| Clr-carbopol | 786 | -28,76 | -35,1 | 0,602 |

### Example 7 - Study of antiviral activity against viruses of the SARS-COV and H1N1 families of alcoholic gel and body spray containing inclusion compound.

The antiviral activity assay with the products containing the formulations described above was conducted following the recommendations of ANVISA Art. 1 and Art. 3 of IN 04/13 and IN 12/16 and methodologies described in the standards (EN14476:2019, ASTM E1053 - 11, and the Robert Koch Institute - RKI).

The titration of the coronavirus (MHV-3 strain) and Influenza Virus (H1N1) was carried out according to the method of infectious dose in 50% tissue cultures (TCID50) against L-929 cells, derived from the L strain (ATCC CCL-1), and MDCK (Madin-Darby Canine Kidney) cells (ATCC CCL-34).

Sequential tenfold dilutions of the virus were performed in quadruplicate in sterile 96-well microplates. Then, L929 and MDCK cells were added at a concentration of 2 × 10⁵ cells/well. After 48 hours, the cytopathic effect (CPE) of viral infection was observed compared to the cell control and viral control.

The products were mixed with the viruses and then subjected to different contact times. Subsequently, they were inoculated into permissive L929 and MDCK cells. The microplates containing the products, viruses, and cell systems were incubated at 37°C in a 5% CO2 incubator for 48 hours. Titers were calculated based on the Reed and Muench method (Reed LJ, Muench H., A simple method of estimating fifty per cent endpoints. Am. J. Hyg. 27, 493-497, 1938).

The results show that the body spray exhibited antiviral activity ranging from 99% to 99.99% after 8 hours of application. The non-alcoholic gel showed viral inhibition of 99.99% immediately and 5 minutes after application, and 99.9% at 2 to 8 hours after application. Meanwhile, the alcoholic gel exhibited activity ranging from 99% to 99.9% against the Coronavirus (MHV-3 strain) and Influenza Virus (H1N1) regardless of the application time.

### Example 8 - Sensory study of solutions at various concentrations of chlorhexidine digluconate and inclusion compound

Sensory evaluation of the reduction in the characteristic bitterness of chlorhexidine was performed using solutions at equivalent concentrations of pure chlorhexidine digluconate and chlorhexidine digluconate encapsulated in beta-cyclodextrin. The studies were conducted by distributing 20 mL of each solution in plastic cups - indiscriminately to the evaluators. After the use of the solutions, a questionnaire was distributed to the participants, inquiring about the taste and bitterness of the products.

All participants described that the taste of the solution containing the inclusion compound was more pleasant and less bitter compared to the pure chlorhexidine digluconate solution at all tested concentrations.

## Claims

1. **LONG-LASTING ANTIMICROBIAL COMPOSITIONS, characterized by** comprising cationic actives selected from the group consisting of chlorhexidine digluconate, ammonium quaternary, or cetyl pyridinium, nanoencapsulated in cyclodextrin, selected from the group consisting of alpha-cyclodextrin, beta-cyclodextrin, gamma-cyclodextrin, hydroxyethyl-beta-cyclodextrin, and hydroxypropyl-beta-cyclodextrin, which may optionally contain alkyl or aryl group insertions, and anionic excipients, in addition to humectants, stabilizers, chelating agents, surfactants, preservatives, or thickeners.

2. **LONG-LASTING ANTIMICROBIAL COMPOSITIONS, according to claim 1, characterized by** the cationic actives being in the molar ratio of 1:1 to 1:4 in relation to cyclodextrin.

3. **LONG-LASTING ANTIMICROBIAL COMPOSITIONS, according to claim 1, characterized by** the pharmaceutical anionic excipient being the anionic surfactant sodium lauryl sulfate in concentrations between 0.01 and 3.0% v/v.

4. **LONG-LASTING ANTIMICROBIAL COMPOSITIONS, according to claim 1 to 3, characterized by** comprehending the thickener carbomer in concentrations between 0.1 and 3.0% w/v.

5. **USE OF THE ANTIMICROBIAL COMPOSITIONS defined in any one of claims 1 to 4, characterized by** to be used in the production of sanitizers in aqueous solution or alcoholic solution, in wet gels, xerogels, aerosols, sprays, powders, polymeric systems, and in solid state.

6. **USE OF THE ANTIMICROBIAL COMPOSITIONS defined in any one of claims 1 to 4, characterized by** to be used in the production of transparent gel.

7. **USE OF THE ANTIMICROBIAL COMPOSITIONS defined in any one of claims 1 to 4, characterized by** to be used in the production of formulations for topical use.

8. **USE OF THE ANTIMICROBIAL COMPOSITIONS defined in any one of claims 1 to 4, characterized by** to be used in the production of formulations for environmental treatment and surface disinfection.

9. **USE OF THE ANTIMICROBIAL COMPOSITIONS defined in any one of claims 1 to 4, characterized by** to be used in the production of mouthwash and toothpaste.

## Amended claims

### Amended claims under Art. 19.1 PCT

1. **LONG-LASTING ANTIMICROBIAL COMPOSITIONS, characterized by** comprising the cationic agent chlorhexidine, nanoencapsulated in cyclodextrin; in a ratio of active ingredient to cyclodextrin between 1:1 and 1:4 (mol:mol), selected from the group consisting of alpha-cyclodextrin, beta-cyclodextrin, gamma-cyclodextrin, hydroxyethyl-beta-cyclodextrin, and hydroxypropyl-beta-cyclodextrin, and anionic excipients at a concentration between 0.01% and 3.0% (v:v).

2. **LONG-LASTING ANTIMICROBIAL COMPOSITIONS, according to claim 1, characterized by** the anionic excipients being the anionic surfactant sodium lauryl sulfate or the thickener carbomer.

3. **USE OF THE ANTIMICROBIAL COMPOSITIONS defined in any of claims 1 and 2, characterized by** being in the production of sanitizers in aqueous solution or alcoholic solution, in wet gels, xerogels, aerosols, sprays, powders, polymeric systems, and in solid form.

4. **USE OF THE ANTIMICROBIAL COMPOSITIONS defined in any of claims 1 and 2, characterized by** being in the production of transparent gels.

5. **USE OF THE ANTIMICROBIAL COMPOSITIONS defined in any of claims 1 and 2, characterized by** being for the production of formulations for topical use.

6. **USE OF THE ANTIMICROBIAL COMPOSITIONS defined in any of claims 1 and 2, characterized by** being for the production of formulations for environment treatment and surface cleaning.

7. **USE OF THE ANTIMICROBIAL COMPOSITIONS defined in any of claims 1 and 2, characterized by** being for the production of mouthwash and toothpaste.
